(19) Europäisches Patentamt
European Patent Office
Office européen des brevets

(11) Veröffentlichungsnummer : **0 129 148**
**B1**

(12) # EUROPÄISCHE PATENTSCHRIFT

(45) Veröffentlichungstag der Patentschrift : **30.12.86**

(51) Int. Cl.⁴ : **C 07 D487/04**, C 08 L 69/00

(21) Anmeldenummer : **84106447.0**

(22) Anmeldetag : **06.06.84**

(54) **Neue Flammschutzmittel, ihre Herstellung und ihre Verwendung zur Flammfestausrüstung in Polycarbonat.**

(30) Priorität : **18.06.83 DE 3322057**

(43) Veröffentlichungstag der Anmeldung : **27.12.84 Patentblatt 84/52**

(45) Bekanntmachung des Hinweises auf die Patenterteilung : **30.12.86 Patentblatt 86/52**

(84) Benannte Vertragsstaaten : **BE DE FR GB IT NL**

(56) Entgegenhaltungen :
**EP-A- 0 010 440**
**EP-A- 0 043 997**
**DE-A- 2 460 937**

(73) Patentinhaber : **BAYER AG**
**Konzernverwaltung RP Patentabteilung**
**D-5090 Leverkusen 1 Bayerwerk (DE)**

(72) Erfinder : **Krishnan, Sivaram, Dr.**
**1653 Little Meadow Road**
**Pittsburgh, PA 15241 (US)**
Erfinder : **Kircher, Klaus, Dr.**
**Alfred-Kubin-Strasse 3**
**D-5090 Leverkusen (DE)**
Erfinder : **Kress, Hans-Jürgen, Dr.**
**Scheiblerstrasse 111**
**D-4150 Krefeld 1 (DE)**

EP 0 129 148 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der vorliegenden Erfindung sind Bis-(imid-sulfonate) der Formel (I)

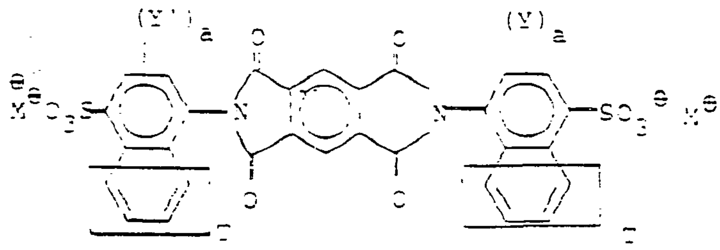

(I)

worin

Y und Y' unabhängig voneinander ein niederer Alkyl-Rest mit 1 bis 4 Kohlenstoffatomen sind,
a für 0 oder eine ganze Zahl von 1 bis 4,
M für ein Alkalimetall und
T für 0 oder 1 stehen.

Geeignete Alkylgruppen sind Methyl-, Ethyl, n-Propyl, Isopropyl-, n-Butyl-, Isobutyl-Gruppen ; geeignete Alkalimetalle sind Natrium oder Kalium.

Die Herstellung der Bis-(imid-sulfonate) der Formel (I) erfolgt durch Reaktion des Benzol-1,2,4,5-tetracarbonsäureanhydrids mit der entsprechenden Aminosulfonsäure der allgemeinen Formel (II) im Molverhältnis 1 zu 2 in Gegenwart von Natrium- bzw. Kaliumcarbonat, das in äquivalenten Mengen, bezogen auf Sulfonsäuren (II), verwendet wird, bei Temperaturen von 130-150 °C und unter Mitverwendung von Dimethylformamid. In den Sulfonsäuren der Formel (II)

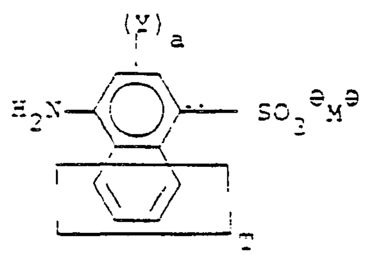

(II)

entsprechen Y, a und T der für Formel (I) genannten Bedeutung.

Gegenstand der vorliegenden Erfindung ist somit außerdem ein Verfahren zur Herstellung der Bis-(imid-sulfonate) der Formel (I), das dadurch gekennzeichnet ist, daß man Benzol-1,2,4,5-tetracarbonsäure-bis-anhydrid mit einer Aminosulfonsäure der Formel (II)

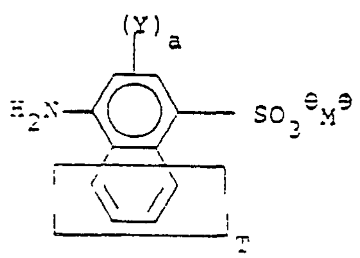

(II)

worin Y, a, M und T die für Formel (I) genannte Bedeutung haben, im Molverhältnis Bis-anhydrid zu Aminosulfonsäure wie 1 zu 2 bei Temperaturen von 130 °C bis 150 °C und unter Mitverwendung von Dimethylformamid, in Gegenwart von äquivalenten Mengen Natriumcarbonat oder Kaliumcarbonat, bezogen auf die jeweilige Menge an Aminosulfonsäure der Formel (II), umsetzt.

Die neuen Bis-(imid-sulfonate) der Formel (I) sind geeignet zur Flammfestausrüstung von aromatischen, thermoplastischen Polycarbonaten, wobei diese in Mengen von 0,01 bis 1 Gew.-%, bezogen auf das Gewicht des Polycarbonatharzes, vorzugsweise in Mengen von 0,1 bis 0,75 Gew.-%, bezogen auf das Gewicht des Polycarbonatharzes, eingesetzt werden.

Ein weiterer Gegenstand der vorliegenden Erfindung ist somit die Verwendung der Bis-(imid-sulfonate) der Formel (I) zur Flammfestausrüstung von aromatischen, thermoplastischen Polycarbonaten aus halogenfreien Diphenolen.

Bekannt sind Flammverzögerungsmittel für Polycarbonate, die der nachfolgenden Formel (III)

entsprechen

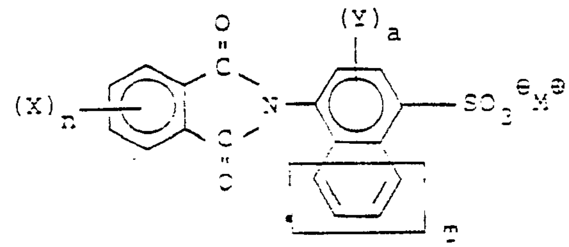

(III)

worin

Y, a, T und M die für Formel (I) genannte Bedeutung haben, und worin

X ein Halogen ist und

n eine ganze Zahl von 1 bis 4 ist.

(Siehe Europäische Offenlegungsschrift 43 997 bzw. US-Patent 4 320 049).

Gegenüber diesen bekannten Flammschutzmitteln besitzen die erfindungsgemäßen Bis-(imid-sulfonate) der Formel (I) die Wirkung, daß sie, obwohl sie keinerlei Halogen im Molekül tragen, eine flammschützende Wirkung aufweisen und somit eine halogenfreies flammgeschütztes Polycarbonat mit einer UL-94-V-O-Klassifizierung ergeben.

Das gemäß US-PS 3 951 910 bzw. DE-OS 2 461 144 als Flammschutzmittel für Polycarbonate genannte Lithium-N-phenyl-phthalimid-4-sulfonat bewirkt selbst in Mengen von 1 Gewichtsprozent in linearen Bisphenol-A-Polycarbonaten nur SE-II-Klassifizierung.

Zusätzlich sind die Bis(imid-sulfonate) der Formel (I) durch niedrige Flüchtigkeit bei normalen Polycarbonat- Verarbeitungsbedingungen gekennzeichnet.

Aromatische, thermoplastische Polycarbonate im Sinne der vorliegenden Erfindung sind durch Umsetzung von halogenfreien Diphenolen, insbesondere von Dihydroxydiarylalkanen, mit Phosgen oder Diestern der Kohlensäure erhältliche Polykondensate, wobei außer den unsubstituierten Dihydroxydiarylalkanen auch solche geeignet sind, deren Arylreste in o- und/oder m-Stellung zur Hydroxylgruppe, Alkylgruppe tragen. Ebenso sind verzweigte Polycarbonate geeignet, beispielsweise die verzweigten gemäß US-Patent 4 185 009 bzw. DE-PS 25 00 092.

Die aromatischen, thermoplastischen Polycarbonate haben mittlere Gewichtsmittel-Molekulargewichte $\bar{M}w$ zwischen 10 000 und 200 000, vorzugsweise zwischen 20 000 und 80 000, ermittelt durch Messung der rel. Viskosität in $CH_2Cl_2$ bei 25 °C und einer Konzentration von 0,5 g/100 ml nach entsprechender Eichung.

Geeignete halogenfreie Diphenole sind z. B. Hydrochinon, Resorcin, 4,4'-Dihydroxydiphenyl, Bis-(hydroxy-phenyl)-alkane wie beispielsweise $C_1$-$C_8$-Alkylen- bzw. $C_2$-$C_8$-Alkylidenbisphenole, Bis-(hydroxyphenyl)-cycloalkane wie beispielsweise $C_5$-$C_{15}$-Cycloalkylen- bzw. $C_5$-$C_{15}$-Cycloalkylidenbisphenole, Bis-(hydroxy-phenyl)-sulfide, -ether, -ketone, -sulfoxide oder -sulfone, ferner α,α'-Bis-(hydroxyphenyl)-diisopropylbenzol sowie die entsprechenden kernalkylierten Verbindungen. Bevorzugt sind Polycarbonate auf Basis Bis-(4-hydroxy-phenyl)-propan-2.2 (Bisphenol A), Bis-(4-hydroxy-3,5-dimethyl-phenyl)-propan-2.2 (Tetramethylbisphenol A), Bis-(4-hydroxy-phenyl)-cyclohexan-1.1 (Bisphenol 2) sowie auf Basis von Dreikernbisphenolen wie α,α'-Bis-(4-hydroxyphenyl)-p-diisopropylbenzol.

Weitere für die Herstellung der Polycarbonate geeignete halogenfreie Diphenole sind in den US-Patenten 3 028 365 und 3 275 601 beschrieben.

Geeignete Verzweiger sind beispielsweise 1,3,5-Tri-(4-hydroxyphenyl)-benzol, Tri-(4-hydroxyphenyl)-phenyl-methan, Hexa-(4-(4-hydroxyphenylisopropyl)-phenyl)-ortho-terephthalsäureester, Tetra-(4-hydroxyphenyl)-methan, 1,4-Bis-(4',4''-dihydroxytriphenyl-methyl)-benzol, Trimesinsäure, Cyanurchlorid und 3,3-Bis-(4-hydroxy-3-methyl-phenyl)-2-oxo-2,3-dihydroindol.

Geeignete Kettenabbrecher zur Regulierung des Molekulargewichts sind beispielsweise in bekannter Weise Phenol und Alkylphenole.

Die Herstellung der aromatischen, thermoplastischen Polycarbonate erfolgt in bekannter Weise beispielsweise nach dem Phasengrenzflächenverfahren oder nach dem Verfahren in homogener Lösung. Auch nach dem bekannten Umesterungsverfahren können die aromatischen, thermoplastischen Polycarbonate hergestellt sein.

Bevorzugte Polycarbonate im Sinne der vorliegenden Erfindung sind bei alleinigem Einsatz des Bis-(imid-sulfonats) der Formel (I) verzweigte Polycarbonate auf Basis Bisphenol-A mit einem Verzweigergehalt von 0,3 bis 1,0 Mol-%, bezogen auf Mole Bisphenol-A ; bei Zusatz weiterer üblicher Flammschutz-Additive wie Polytetrafluorethylenpolymere, Kryolith oder $BaCO_3$ sind auch lineare Polycarbonate auf Basis Bisphenol-A bevorzugte Polycarbonate.

Die Einarbeitung der neuen Flammschutzmittel in die Polycarbonate kann beispielsweise erfolgen durch Mischung und anschließende Granulierung des Materials über einen Doppelwellenextruder bei 270-280 °C.

Die optimalen Verarbeitungsbedingungen sind derart, daß bei einer Umdrehungszahl von 40-80

Umdrehungen/min ein Durchsatz von 18 kg/Stunde erreicht wird.

Der benutzte Doppelwellenextruder ist ein Apparat der Firma « Werner und Pfleiderer » mit der Bezeichnung ZSK 53.

Gegenstand der vorliegenden Erfindung ist somit auch ein Verfahren zur Flammfestausrüstung von aromatischen, thermoplastischen Polycarbonaten aus halogenfreien Diphenolen, das dadurch gekennzeichnet ist, daß man Bis-(imid-sulfonate) der Formel (I) in Mengen von 0,01 bis 1 Gew.-%, vorzugsweise von 0,1 bis 0,75 Gew.-%, bezogen auf aromatisches, thermoplastisches Polycarbonat, gegebenenfalls in Gegenwart weiterer Zusätze, in aromatische, thermoplastische Polycarbonate aus halogenfreien Diphenolen über einen Doppelwellenextruder einarbeitet, wobei eine Temperatur von 270-280 °C sowie ein Durchsatz von 18 kg/Stunde bei einer Umdrehungszahl von 40-80 Umdrehungen/min, insbesondere von 60 Umdrehungen/min eingehalten wird.

Ein weiterer Gegenstand der Erfindung sind die aromatischen, thermoplastischen Polycarbonate aus halogenfreien Diphenolen mit einem Gehalt von 0,01 bis 1 Gew.-%, vorzugsweise 0,1 bis 0,75 Gew.-%, bezogen auf aromatisches thermoplastisches Polycarbonat, an Bis-(imid-sulfonat) der Formel (I).

Den erfindungsgemäßen Abmischungen aus Polycarbonat und Bis-(imid-sulfonat) der Formel (I) können in bekannter Weise (siehe beispielsweise DE-OS 25 35 262) Polytetrafluorethylen-Polymere zusätzlich zugesetzt enthalten, wobei sowohl faserbildende als auch nicht-faserbildende Polytetrafluorethylen-Polymere eingesetzt werden können. Die Menge an einzusetzenden Polytetrafluorethylen-Polymeren soll zwischen 0,01 und 3 Gew.-%, vorzugsweise zwischen 0,01 und 2 Gew.-%, bezogen auf aromatisches, thermoplastisches Polycarbonat, betragen.

Die erfindungsgemäßen Abmischungen aus Polycarbonat und Bis-(imid-sulfonat) der Formel (I) können noch andere in der Polycarbonatchemie übliche Additive enthalten, wie beispielsweise Pigmente, Farbstoffe, Füllstoffe, Stabilisatoren oder Entformungsmittel.

Die erfindungsgemäßen Abmischungen können zu Formteilen oder Folien verarbeitet werden.

Die Herstellung von Formkörpern erfolgt nach dem Spritzgießverfahren bei einer Temperatur von 300-310 °C.

Die erfindungsgemäßen Abmischungen können eingesetzt werden z. B. im Elektrobereich für Schalterblenden, Steckdosen, Steckerleisten, Schaltkästen usw., im Haushaltssektor für Gehäuseteile von Bügeleisen, Kaffeemaschinen und im Großgerätebereich z. B. für Computergehäuseteile.

Die erfindungsgemäßen Abmischungen aus Polycarbonat und Bis-(imid-sulfonat) der Formel (I) besitzen eine Flammwidrigkeit die gemäß UL-94-V-Test als V-O bezeichnet wird.

Nach dem UL-94-Test (Underwriters' Laboratories, Inc.) werden Polycarbonatproben zu Stäben der Abmessungen 127 mm × 12,7 mm × 1,6 (oder 3,2) mm (5,00 inch × 0,5 inch × 1/16 (oder 1/8) inch) geformt. Die Stäbe werden vertikal so montiert, daß die Unterseite des Probekörpers sich 305 mm über einen Streifen Verbandsstoff befindet. Jeder Probestab wird einzeln mittels zweier aufeinanderfolgender Zündvorgänge von 10 s Dauer entzündet, die Brenneigenschaften nach jedem Zündvorgang werden beobachtet und danach die Probe bewertet. Zum Entzünden der Probe wird ein Bunsenbrenner mit einer 10 mm (3/8 inch) hohen blauen Flamme von Erdgas mit einem Wärmeinhalt von $3{,}73 \times 10^4$ kJ/$m^3$ (1,000 BTU per cubic foot) benutzt.

Die UL 94 V-O Klassifizierung umfaßt die nachstehend beschriebenen Eigenschaften von Materialien, die gemäß der UL-94-Vorschrift geprüft wurden. Die Polycarbonate in dieser Klasse enthalten keine Proben, die länger als 10 s nach jeder Einwirkung der Testflamme brennen ; sie zeigen keine Gesamt-Flammzeit von mehr als 50 s bei der zweimaligen Flammeneinwirkung auf jeden Probensatz ; sie enthalten keine Proben, die vollständig bis hinaus zu der am oberen Ende der Probe befestigten Halteklammer abbrennen ; sie weisen keine Proben auf, die die unterhalb der Probe angeordnete Watte durch brennende Tropfen oder Teilchen entzünden ; sie enthalten auch keine Proben, die länger als 30 s nach Entfernen der Testflamme glimmen.

Andere UL 94-Klassifizierungen bezeichnen Proben, die weniger flammwidrig und selbstverlöschend sind und die flammende Tropfen oder Teilchen abgeben. Diese Klassifizierungen werden mit UL 94 V-1 und V-2 bezeichnet. Die Polycarbonate innerhalb des Bereichs dieser Erfindung zeigen in charakteristischer Weise die für eine UL 94 V-O-Klassifizierung geforderten Eigenschaften.

## Beispiele

### A. Herstellung von Bis-(imid-sulfonaten) der Formel (I)

$A_1$. N,N'-Bis-(4-natriumsulfonylphenyl)-pyromellithsäurediimid

0,6 Mol (103,8 g) 4-Aminobenzolsulfonsäure und 0,3 Mol (31,8 g) Natriumcarbonat werden in 2 500 ml Dimethylformamid vorgelegt. Die Reaktionsmischung ergibt bei 128 °C eine gelbe Lösung. Zu dieser werden 0,3 Mol (65,4 g) Pyromellithsäureanhydrid mit einer geringen Menge Dimethylformamid gegeben. Hierbei fällt ein gelber Niederschlag aus. Die Reaktionsmischung wird für drei Stunden auf 146 °C gebracht und anschließend abgekühlt. Die ausgefallenen hellgelben Kristalle werden abgesaugt und mit ca. 7 l heißem dest. Wasser aufgeschlämmt. Hierauf wird der restliche Niederschlag abgesaugt und unter einem Wasserstrahlvakuum bei 110 °C getrocknet.

Ausbeute : 148 g ; Natriumgehalt :
theor. : 8,04 %
gef. : 8,0-7,9 %

$A_2$. N,N'-Bis-(4-kaliumsulfonylphenyl)-p-pyromellithsäurediimid

Abweichend von Beispiel $A_1$ wird hier Kaliumcarbonat bei sonst gleicher Reaktionsführung eingesetzt.

B.

$B_1$. Ein verzweigtes Polycarbonat auf Basis Bisphenol A, 0,5 Mol-% 3,3-Bis-(4-hydroxy-3-methylphenyl)-2-oxo-2,3-dihydroindol, 3,0 Mol-% Phenol als Kettenabbrecher und Phosgen mit einer Lösungsviskosität von 1,31 (gemessen in $CH_2Cl_2$ bei 25 °C und in einer Konzentration von 0,5 g pro 100 ml) wurde mit N,N'-Bis-(4-natriumsulfonylphenyl)-pyromellithsäure-diimid ($A_1$) vermischt, extrudiert und gemäß UL-94 in einer Dicke von 3,2 mm (1/8") auf seine Brandwidrigkeit untersucht. Ergebnis : UL-94 = V-O.

$B_2$-$B_4$.

In Abänderung von Beispiel 1 wurde ein lineares Polycarbonat verwendet und zusätzlich zu $A_1$ weitere Komponenten mit Polycarbonat vermischt und extrudiert. Diese Proben wurden in verschiedenen Wandstärken nach UL-94 auf ihre Brandwidrigkeit hin untersucht. In Beispiel 4 ist als wirksame Komponente Bis-(4-kaliumsulfonylphenyl)-pyromellithsäurediimid ($A_2$) eingesetzt worden. Die genaue Zusammensetzung sowie die erhaltenen Branddaten sind folgender Tabelle zu entnehmen.

(Siehe Tabelle 1 Seite 6 f.)

Tabelle 1

| | Polycarbonat* % | $A_1$ % | $A_2$ % | Polytetrafluorethylen ** % | Kyrolith % | $BaCO_3$ % |
|---|---|---|---|---|---|---|
| Beispiel $B_2$ | 99,35 | 0,5 | - | 0,15 | - | - |
| UL-94 3,2 mm | V-O | | | | | |
| 1,6 mm | V-O | | | | | |
| Beispiel $B_3$ | 98,23 | 0,75 | - | 0,15 | 0,37 | 0,5 |
| UL-94 3,2 mm | V-O | | | | | |
| 1,6 mm | V-O | | | | | |
| 0,8 mm | V-1 | | | | | |
| Beispiel $B_4$ | 98,23 | - | 0,75 | 0,15 | 0,37 | 0,5 |
| UL-94 3,2 mm | V-O | | | | | |
| 1,6 mm | V-O | | | | | |
| 0,8 mm | V-1 | | | | | |

* Lineares Polycarbonat auf Basis Bispienol-A Phenol und Phosgen mit einem $\eta$ rel = 1,29, gemessen in $CH_2Cl_2$ bei 25 °C als 0,5 g pro 100 ml

** Polytetrafluorethylen hat eine mittlere Teilchengröße im Bereich von 500 bis 650 µm und eine Dichte von 2.17-2.19 g/cm³.

**Patentansprüche**

1. Bis-(imid-sulfonate) der Formel (I)

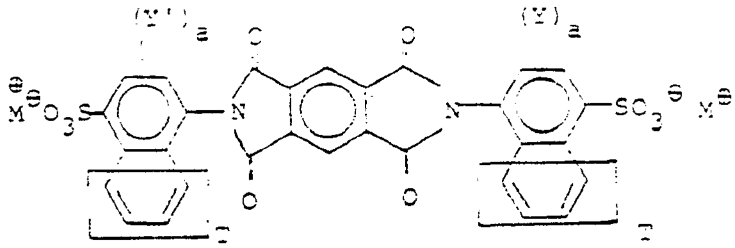

(I)

worin

Y und Y' unabhängig voneinander ein niederer Alkylrest mit 1 bis 4 Kohlenstoffatomen sind,
a für 0 oder eine ganze Zahl von 1 bis 4,
M für ein Alkalimetall und
T für 0 oder 1 stehen.

2. Verfahren zur Herstellung der Bis-(imid-sulfonate) der Formel (I), dadurch gekennzeichnet, daß man Benzol-1,2,4,5-tetracarbonsäure-bis-anhydrid mit einer Aminosulfonsäure der Formel (II)

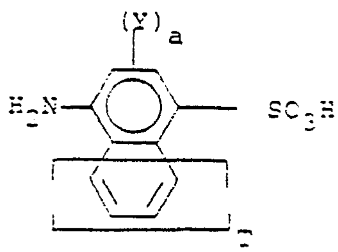

(II)

worin Y, a, M und T die für Formel (I) genannte Bedeutung haben, im Molverhältnis Bis-anhydrid zu Aminosulfonsäure wie 1 : 2 bei Temperaturen von 130 °C bis 150 °C und unter Mitverwendung von Dimethylformamid in Gegenwart von äquivalenten Mengen Natriumcarbonat oder Kaliumcarbonat, bezogen auf die jeweilige Menge an Aminosulfonsäure der Formel (II), umsetzt.

3. Verwendung der Bis-(imid-sulfonate) der Formel (I) des Anspruchs 1 zur Flammfestausrüstung von aromatischen, thermoplastischen Polycarbonaten aus halogenfreien Diphenolen.

4. Verfahren zur Flammfestausrüstung von aromatischen, thermoplastischen Polycarbonaten aus halogenfreien Diphenolen, dadurch gekennzeichnet, daß man Bis-(imid-sulfonate) der Formel (I) des Anspruchs 1 in Mengen von 0,01 bis 1 Gew.-%, bezogen auf aromatisches, thermoplastisches Polycarbonat, gegebenenfalls in Gegenwart weiterer Zusätze, in aromatische thermoplastische Polycarbonate aus halogenfreien Diphenolen über einen Doppelwellenextruder einarbeitet, wobei eine Temperatur von 270-280 °C, sowie einem Durchsatz von 18 kg/Stunde bei einer Umdrehungszahl von 40-80 Umdrehungen/min eingehalten wird.

5. Thermoplastische, aromatische Polycarbonate aus halogenfreien Diphenolen mit einem Gehalt von 0,01 bis 1 Gew.-%, bezogen auf aromatisches, thermoplastisches Polycarbonat, an Bis-(imid-sulfonat) der Formel (I) des Anspruchs 1.

**Claims**

1. Bis-(imid-sulphonates) of the formula (I)

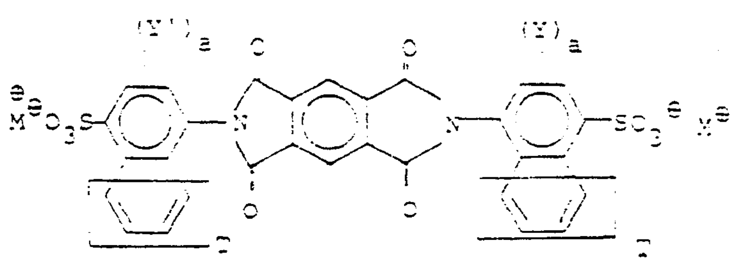

(I)

wherein

Y and Y' independently of one another are
a lower alkyl radical with 1 to 4 carbon atoms,
a represents 0 or an integer from 1 to 4,
M represents an alkali metal and
T represents 0 or 1.

2. Process for the preparation of the bis-(imide-sulphonates) of the formula (I), characterised in that benzene-1,2,3,4,5-tetracarboxylic acid bis-anhydride is reacted with an aminosulphonic acid of the formula (II)

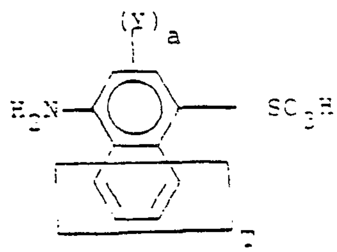

(II)

wherein Y, a, M and T have the meaning given for formula (I), in a molar ratio of bis-anhydride to aminosulphonic acid of 1 : 2 at temperatures of 130 °C to 150 °C, dimethylformamide also being used, in the presence of equivalent amounts of sodium carbonate or potassium carbonate, based on the particular amount of aminosulphonic acid of the formula (II).

3. Use of the bis-(imide-sulphonates) of the formula (I) of Claim 1 for flameproofing aromatic, thermoplastic polycarbonates obtained from halogen-free diphenols.

4. Process for flameproofing aromatic, thermoplastic polycarbonates obtained from halogen-free diphenols, characterised in that bis-(imide-sulphonates) of the formula (I) of Claim 1 are incorporated into aromatic, thermoplastic polycarbonates, which are obtained from halogen-free diphenols, in amounts of 0.01 to 1 % by weight, based on the aromatic, thermoplastic polycarbonate, if appropriate in the presence of other additives, via a twin-screw extruder, a temperature of 270-280 °C and a throughput of 18 kg/hour at a rotation speed of 40-80 revolutions/minute being maintained.

5. Thermoplastic, aromatic polycarbonates which are obtained from halogen-free diphenols and contain 0.01 to 1 % by weight, based on the aromatic, thermoplastic polycarbonate, of bis-(imide-sulphonate) of the formula (I) of Claim 1.

## Revendications

1. Bis-(imidosulfonates) de formule (I)

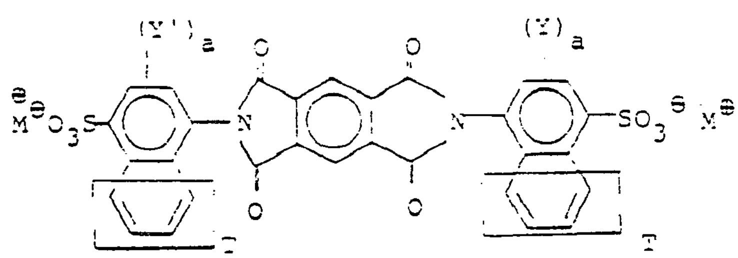

(I)

dans laquelle

Y et Y' représentent, indépendamment l'un de l'autre, un reste alkyle inférieur ayant 1 à 4 atomes de carbone,
a est égal à 0 ou à un nombre entier de 1 à 4
M est un métal alcalin et
T a la valeur 0 ou 1.

2. Procédé de production de bis-(imidosulfonates) de formule (I), caractérisé en ce qu'on fait réagir le bis-anhydride d'acide benzène-1,2,4,5-tétracarboxylique avec un acide aminosulfonique de formule (II)

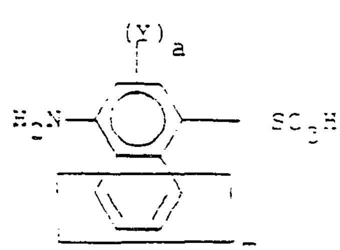

(II)

dans laquelle Y, a, M et T ont la définition mentionnée pour la formule (I), dans un rapport molaire du bis-anhydride à l'acide aminosulfonique de 1 : 2 à des températures de 130 °C à 150 °C et en utilisant en même temps du diméthylformamide en présence de quantités équivalentes de carbonate de sodium ou de carbonate de potassium, par rapport à la quantité respective d'acide aminosulfonique de formule (II).

3. Utilisation des bis-(imidosulfonates) de formule (I) suivant la revendication 1 pour l'ignifugeage de polycarbonates thermoplastiques aromatiques dérivés de diphénols non halogénés.

4. Procédé pour l'ignifugeage de polycarbonates aromatiques thermoplastiques dérivés de diphénols non halogénés, caractérisé en ce qu'on incorpore des bis-(imidosulfonates) de formule (I) suivant la revendication 1 en quantités de 0,01 à 1 % en poids, par rapport au polycarbonate aromatique thermoplastique, le cas échéant en présence d'autres additifs, à des polycarbonates aromatiques thermoplastiques dérivés de diphénols non halogénés, au moyen d'une extrudeuse à deux arbres, en maintenant une température de 270-280 °C ainsi qu'un débit de 18 kg/heure pour une vitesse de rotation de 40 à 80 tr/min.

5. Polycarbonates aromatiques thermoplastiques dérivés de diphénols non halogénés, ayant une teneur de 0,01 à 1 % en poids, par rapport au polycarbonate aromatique thermoplastique, de bis-(imidosulfonate) de formule (I) suivant la revendication 1.